# EUROPÄISCHE PATENTANMELDUNG

(11) **EP 3 479 849 A1**
(43) Veröffentlichungstag der Anmeldung: **08.05.2019**
(21) Anmeldenummer: 17199671.3
(22) Anmeldetag: 02.11.2017
(51) Int. Cl.: A61L 2/10, A61M 1/36

(54) **VERFAHREN UND VORRICHTUNG ZUR INAKTIVIERUNG VON PATHOGENEN**

(71) Anmelder: Justus-Liebig-Universität Gießen, 35390 Gießen (DE)
(72) Erfinder: Misterek, Hans-Joachim, 35435 Wettenberg (DE); Prof. Dr. Bein, Gregor, 35394 Gießen (DE)
(74) Vertreter: Stumpf, Peter

(57) **Zusammenfassung**

Die vorliegende Erfindung offenbart ein Verfahren sowie eine Vorrichtung zur Inaktivierung von Pathogenen in Flüssigkeiten, die dazu bestimmt sind, in den menschlichen oder tierischen Körper infundiert zu werden, z. B. Thrombozytenkonzentrate. Die zu behandelnde Flüssigkeit wird an mindestens einer UV-Strahlungsquelle entlanggeführt wird, so dass die Pathogen-Inaktivierung durch Bestrahlung in einem Durchflußverfahren erfolgt. Es können zahlreiche Einzelproben kosten- und zeitsparend parallel bestrahlt werden. Alternativ kann das Durchfluß-Belichtungsverfahren auch großtechnisch als inline-Verfahren eingesetzt werden.

## Beschreibung

Die vorliegende Erfindung betrifft ein Verfahren und eine Vorrichtung zur Inaktivierung von Pathogenen in Flüssigkeiten, die dazu bestimmt sind, in den menschlichen oder tierischen Körper infundiert zu werden.

### Stand der Technik

Flüssigkeiten, die in den menschlichen oder tierischen Organismus injiziert bzw. infundiert werden sollen, insbesondere Blutprodukte (und unter diesen auch solche, die äußerlich angewendet werden, wie beispielsweise Serum-Augentropfen), müssen möglichst frei von Pathogenen jeglicher Art sein, um Schädigungen des Organismus zu vermeiden. Bei großtechnisch hergestellten Infusionslösungen, wie beispielsweise isotonischer Kochsalzlösung gelingt dies sehr einfach durch Sterilisation. Für Blutprodukte, wie beispielsweise Vollblut, Thrombozytenkonzentrate (TK) oder Blutplasma können die sonst zum Einsatz gelangenden Verfahren oftmals leider nicht angewendet werden, da wichtige Bestandteile dadurch zerstört würden. Besonders problematisch ist die Handhabung von Thrombozytenkonzentraten, da diese nicht gekühlt gelagert werden können. Hier ist daher die Gefahr besonders groß, dass sich in dem Thrombozytenkonzentrat befindliche pathogene Keime unter den nahezu idealen Wachstumsbedingungen bei der Lagertemperatur von ca. 20 °C vermehren und das Thrombozytenkonzentrat damit unbrauchbar machen.

Blutprodukte, insbesondere Thrombozytenkonzentrate werden daher nach ihrer Gewinnung aus Vollblut in Portionsbeutel abgefüllt, aus denen sie später appliziert werden, und darin einer UV-Bestrahlung unterworfen. Da die UV-Strahlung nur eine sehr geringe Eindringtiefe von wenigen mm in das zu bestrahlende Thrombozytenkonzentrat hat, müssen diese Beutel während der Bestrahlung bewegt werden, um den Inhalt zu durchmischen und dadurch sicherzustellen, dass das gesamte Volumen bestrahlt wird. Dies führt auch dazu, dass sehr lange bestrahlt werden muß, um sicher sein zu können, dass das gesamte Konzentratvolumen bestrahlt wurde.

Diese Vorgehensweise bei der Bestrahlung mit UV-Licht ist sehr ungenau und schlecht kontrollierbar, da das Strömungsverhalten im Beutel unklar ist (Wellen, Wirbel, unterschiedliche Filmdicke, unterschiedliche Blutzellkonzentrationen). Der zu belichtende Beutel hat in der Regel ein Fassungsvermögen von 1000ml, in dem 200-300ml Thrombozyten- oder Plasmasuspension enthalten sind. Das genaue, tatsächliche, Strömungsverhalten bei einem solchen Vorgang ist unbekannt und von Beutel zu Beutel verschieden, somit völlig undefiniert. Je nach individueller Beschaffenheit der Suspension kann der Bestrahlungsvorgang von Produkt zu Produkt unterschiedlich sein. Um diese Unwägbarkeiten auszugleichen und sicherzustellen, dass der gesamte Beutelinhalt bestrahlt wird, muss eine entsprechend hohe Dosis UV-Strahlung, d.h. eine entsprechend verlängerte Expositionszeit gewählt werden, um jedem möglichen Strömungsverhalten gerecht zu werden. Somit ist es unvermeidlich, dass Zellpopulationen im Produkt zu lange oder mit einer zu hohen Dosis bestrahlt werden, und es somit zur Schädigung der Zellen kommt. Derartige Zellschäden, insbesondere bei Verwendung von UV-C Strahlen sind im Stand der Technik bereits beschrieben. Aber trotz der verlängerten Belichtungsdauer verhindert das durch die Agitation (Bewegung) der Beutel entstehende Strömungschaos in dem Thrombozytenkonzentrat eine sichere Eliminierung aller kontaminierenden Mikroorganismen, so dass man bei den bekannten Verfahren nur von einer Pathogenreduktion sprechen kann.

Die UV-Belichtung erfolgt im Stand der Technik in der Regel etwa 24 Stunden nach Herstellung/Gewinnung des TK. Aggressive Erregerarten können sich in dieser Zeit bereits mehrfach geteilt und in Biofilmen (meistens zwischen Flüssigkeit und Behälterwand) organisiert bzw. Netzwerkstrukturen gebildet haben. Bekanntermaßen sind Mikroorganismen in Biofilmen besonders schwer zu bekämpfen, da kaum auszuschließen ist, dass einzelne Bereiche des Biofilmes bei der Bekämpfung nicht erreicht werden und sich die Mikroorganismen von dort aus nach Beendigung der Maßnahme wieder ausbreiten.

Herkömmliche Belichtungsgeräte, in die - nach Möglichkeit - mehrere Produktbeutel gleichzeitig eingelegt und belichtet werden können, sind sehr groß, nehmen daher in kleineren Einrichtungen zu viel Platz weg, sind teuer in Anschaffung und Unterhalt, sowie personalintensiv und unflexibel in der Anwendung, beispielsweise nicht tragbar und daher nur stationär einsetzbar.

Die im Stand der Technik bekannten Verfahren arbeiten üblicherweise mit UV-A Strahlen oder UV-B Strahlen und erfordern daher den Einsatz eines geeigneten Photosensibilisators, etwa Psoralen oder Riboflavin. Dieser Photosensibilisator muß vor der Verwendung des TK ausgewaschen werden, was einen zusätzlichen aufwendigen Verfahrensschritt darstellt.

Bei den im Stand der Technik bekannten Verfahren kann immer nur ein Produkt (oder nur wenige Produkte/Produktbeutel gemeinsam) bestrahlt werden.

Bekannte Verfahren der vorstehend beschriebenen Art sind das Interceptverfahren der Fa. Cerus und das Mirasol-Verfahren der Fa. TerumoBCT.

### Aufgabe

Aufgabe der vorliegenden Erfindung ist es, die im Stand der Technik bestehenden Nachteile und Unzulänglichkeiten bei der Inaktivierung von Pathogenen zu beseitigen.

### Lösung der Aufgabe

Das erfindungsgemäße Verfahren ermöglicht zur Lösung der Aufgabe:
- eine drastische Reduzierung der erforderlichen Mindestdosierung an UV-Strahlung durch Unterbindung der Biofilmbildung infolge Reduktion des Zeitverzuges in der Anwendung des Verfahrens auf das Produkt;
- eine Vermeidung von Überdosierung der UV-Strahlung;
- eine Verkürzung der Behandlungszeit,
- eine sichere und vollständige Inaktivierung aller Pathogene,
- eine Erhöhung des Mengendurchsatzes und
- eine deutliche Verkleinerung der Vorrichtung zur Inaktivierung von Pathogenen, so dass sie sowohl stationär als auch ambulant einsetzbar ist.

Insgesamt ermöglicht die Erfindung damit eine vollständige Pathogeninaktivierung gegenüber der bisher im Stand der Technik lediglich erreichten Pathogenreduktion, und zwar bei deutlich reduziertem Zeitaufwand.

Diese Aufgabe wird erfindungsgemäß gelöst durch die kennzeichnenden Merkmale des in Anspruch 1 offenbarten Verfahrens sowie der in Anspruch 5 offenbarten Vorrichtung. Die Unteransprüche beschreiben weitere vorteilhafte Ausgestaltungen der Erfindung, wobei diese nicht einschränkend zu verstehen sind. So umfassen beispielsweise Bereichsangaben stets alle - nicht genannten - Zwischenwerte und alle denkbaren Teilintervalle.

Das Verfahren und die Vorrichtung betrifft insbesondere Blutprodukte jeglicher Art; beispielsweise, jedoch nicht einschränkend, seien genannt: Vollblut, Thrombozytenkonzentrat aus Apherese oder gepoolt (d.h. Thrombozytenkonzentrat im Zuge extrakorporaler Zirkulation oder als aus mehreren Spenderproben zusammengemischte Produktcharge), Frischplasma, Erythrozytenkonzentrat, Granulozytenkonzentrat, autologe sowie allogene Serum-Augentropfen. Darüber hinaus betrifft das Verfahren und die Vorrichtung natürlich auch andere biologische Flüssigkeiten für therapeutische und/oder nicht-therapeutische Zwecke.

Insgesamt betreffen das Verfahren und die Vorrichtung somit generell Flüssigkeiten, die Pathogene enthalten können. Als Flüssigkeit im Sinne dieser Offenbarung werden daher auch Suspensionen, Dispersionen, Emulsionen sowie weitere vorstehend nicht genannte Zell-Aufschlämmungen und echte Lösungen verstanden.

Das Verfahren ermöglicht die Zerstörung von Mikroorganismen wie Bakterien, Viren, Pilzen und Parasiten in Flüssigkeiten, insbesondere in Blutprodukten zur Transfusion. Das führt zu einer wesentlich größeren Transfusionssicherheit für den Empfänger des Blutproduktes gegenüber dem aktuellen Stand der Technik. Die Zerstörung bzw. Pathogeninaktivierung erfolgt in der dafür vorgesehenen Vorrichtung durch UV-Licht im laufenden Herstellungsprozess des Blutproduktes oder im Anschluß daran (letzteres analog zum bisherigen Vorgehen im Stand der Technik).

Nachfolgend wird das Verfahren beispielhaft, jedoch nicht einschränkend, anhand von Thrombozytenkonzentrat (TK) beschrieben.

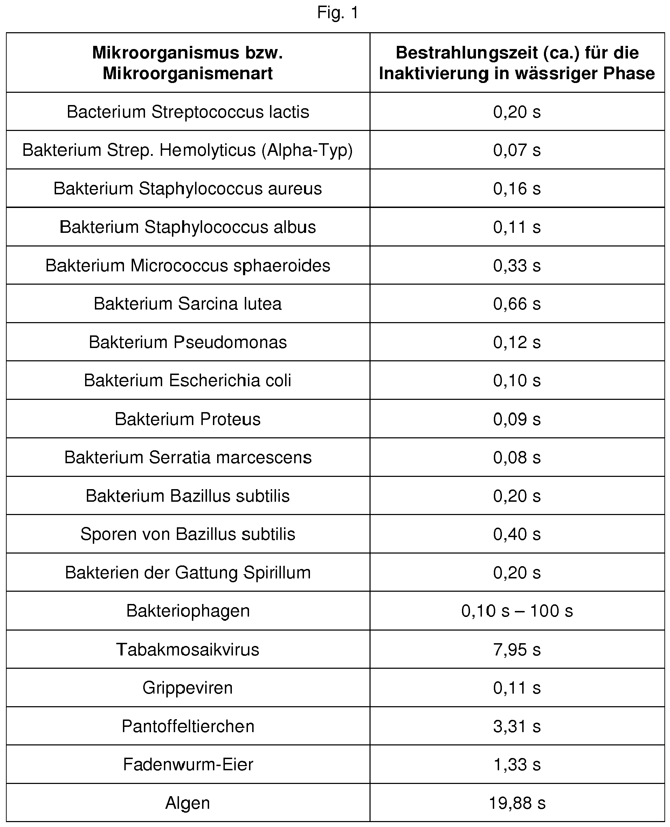

Das Verfahren zur Inaktivierung von Pathogenen in Flüssigkeiten, insbesondere Blutprodukten zeichnet sich dadurch aus, dass die zu bestrahlende Flüssigkeit (Blutprodukt) an mindestens einer UV-Strahlungsquelle in einer variablen oder konstanten Schichtdicke entlanggeführt wird, die maximal der fünffachen, bevorzugt maximal der dreifachen und ganz besonders bevorzugt maximal der doppelten Eindringtiefe der verwendeten UV-Strahlung in die betreffende Flüssigkeit (Blutprodukt) entspricht. In einer besonders bevorzugten Ausführungsform beträgt die maximale Schichtdicke, in der die Flüssigkeit an der Strahlungsquelle entlanggeführt wird, maximal die einfache Eindringtiefe der verwendeten UV-Strahlung, sofern die Belichtung nur von einer Seite erfolgt, und maximal die doppelte Eindringtiefe der verwendeten UV-Strahlung, wenn die Bestrahlung von mehreren Seiten, insbesondere von einander gegenüberliegenden Seiten erfolgt. Es ist darüber hinaus auch erfindungsgemäß, größere Schichtdicken zu verwenden, da auch bei laminarer Strömung infolge Diffusion eine gewisse Vermischung senkrecht zur Fließrichtung stattfindet. Bei Ausgestaltung derart, dass eine turbulente Strömung auftritt, erfolgt diese "Quervermischung" in größerem Umfange durch die Turbulenz, so dass auch größere Schichtdicken verwendet werden können, ohne die Effektivität des Verfahrens zu beeinträchtigen. Die Realisierung entsprechender Quervermischung kann beispielsweise durch Einbau von Strömungshindernissen im verwendeten Strömungskanal oder die Mitführung von Gaseinschlüssen in der Flüssigkeit erfolgen. Die Fließgeschwindigkeit der Flüssigkeit (des Blutprodukts) sowie die Länge der Bestrahlungsstrecke sind so aufeinander abgestimmt, dass eine Bestrahlungszeit erreicht wird, die ausreicht, um die in der jeweiligen Flüssigkeit relevanten Pathogene sicher zu inaktivieren; in Tabelle 1 (Fig. 1) sind Beispielwerte zusammengestellt, anhand derer man sich bei der Auswahl der gewünschten Bestrahlungszeit orientieren kann. Erfindungsgemäß sind Bestrahlungszeiten von 0,01 s bis 1000 s, bevorzugt von 0,1 s bis 100 s und besonders bevorzugt von 1 s bis 100 s, wobei die anzuwendende Bestrahlungszeit auch von der Wellenlänge der verwendeten UV-Strahlung sowie der Strahlungsintensität der Strahlungsquelle abhängig ist. Die zu bestrahlende Flüssigkeit (Blutprodukt) wird in mindestens einem im wesentlichen quadratischen, rechteckigen, rundlichen, ovalen oder hohlzylinderartigen Strömungskanal geführt, wobei dieser mindestens eine Strömungskanal entweder flexibelwandig oder starrwandig aus einem Material ausgeführt ist, das für die verwendete UV-Strahlung durchlässig ist. Bei dem erfindungsgemäßen Verfahren handelt es sich also um ein kontinuierlich arbeitendes Durchflußverfahren. Die Querschnittsgeometrie dieses mindestens einen Strömungskanals kann sehr frei gewählt werden. Wichtig ist, dass nach Möglichkeit das gesamte Innenvolumen des Strömungskanals im gefüllten Zustand, d.h. während die zu behandelnde Flüssigkeit den Strömungskanal durchströmt, von der verwendeten Strahlung erreicht wird. Um den Querschnitt bzw. Durchmesser des Strömungskanals möglichst groß wählen zu können, ist es vorteilhaft, den Strömungskanal von mindestens zwei Seiten, bevorzugt einander gegenüberliegenden Seiten zu bestrahlen. Im Falle des oben beispielhaft genannten hohlzylinderartigen Strömungskanals, bei dem die zu behandelnde Flüssigkeit in einem Spalt zwischen der inneren Hohlzylinderwandung und der äußeren Hohlzylinderwandung strömt, bedeutet dies, dass sich z.B. eine Strahlungsquelle im zentralen Hohlraum des Strömungskanals befindet und/oder mindestens eine weitere Strahlungsquelle außerhalb des Strömungskanals im wesentlichen gegenüber der inneren Strahlungsquelle, so dass die in dem spaltförmigen Zwischenraum des hohlzylinderartigen Strömungskanals befindliche Flüssigkeit von zwei Seiten bestrahlt werden kann. Das Material, aus dem der mindestens eine Strömungskanal besteht, kann starrwandig sein, aber auch flexibelwandig, wobei flexibelwandige Strömungskanäle bevorzugt aus einem geeigneten Polymer gefertigt sind. Dies ermöglicht es vorteilhafterweise, die Strömungskanäle als Einmalmaterial auszubilden, d.h. als Strömungskanäle, die nur ein einziges Mal verwendet werden, was im medizinischen Bereich aus hygienischen Gründen sehr bevorzugt ist. Dadurch werden erfindungsgemäße Anordnungen/Vorrichtungen möglich, wie sie weiter unten als Ausführungsbeispiele anhand der Zeichnungen näher beschrieben sind, ohne dass damit jedoch eine Einschränkung auf die gezeigten Beispiele verbunden ist.

Bei der im Rahmen des erfindungsgemäßen Verfahrens verwendeten UV-Strahlung handelt es sich um UV-A Strahlung, UV-B Strahlung oder UV-C Strahlung. Es ist auch möglich, mehrere Wellenlängenbereiche gleichzeitig zu verwenden, also beispielsweise UV-A Strahlung und UV-B Strahlung oder UV-B Strahlung und UV-C Strahlung. Bei der Wahl des verwendeten Wellenlängenbereiches ist zu beachten, dass es im Falle der Verwendung von UV-A Strahlung oder UV-B Strahlung notwendig ist, einen oder mehrere geeignete Photosensibilisatoren zu verwenden. Bei Verwendung von UV-C Strahlung ist das - aufgrund der höheren Strahlungsenergie - nicht erforderlich. Bevorzugt wird daher UV-C Strahlung verwendet.

Bei dem Material, aus dem der Strömungskanal in dem erfindungsgemäßen Verfahren und der erfindungsgemäßen Vorrichtung besteht, handelt es sich um ein Material, das durchlässig ist für die verwendete UV-Strahlung. Dem Fachmann sind zahlreiche Materialien aus dem Stand der Technik bekannt, die verwendet werden können, ohne den Umfang der Erfindung sowie ihrer Äquivalent zu verlassen. Das bevorzugte Material wird beispielsweise ausgewählt aus der Liste umfassend EVA (Ethylen-Vinylacetat-Copolymere), PVC (Polyvinylchlorid), PE (Polyethylen), Glas (insbesondere UV-durchlässige Spezialgläser), Quarzglas u.a.m., wobei diese Liste nicht einschränkend zu verstehen ist. PE wird beispielsweise vorteilhaft für die Verwendung von UV-B Strahlung verwendet, EVA für die Verwendung von UV-C Strahlung.

Das Verfahren wird bevorzugt angewendet für Flüssigkeiten wie Vollblut oder aus Vollblut gewonnene Flüssigkeiten wie etwa Thrombozytenkonzentrat oder Blutplasma (auch Frischplasma), Erythrozytenkonzentrat, Granulozytenkonzentrat, autologe oder allogene Serum-Augentropfen. Diese Liste ist nicht einschränkend zu verstehen; dem Fachmann sind weitere biologische Flüssigkeiten für therapeutische oder nichttherapeutische Zwecke bekannt, die verwendet werden können, ohne den Umfang der Erfindung oder ihrer Äquivalente zu verlassen.

Die Vorrichtung zur Durchführung des Verfahrens, d.h. die Pathogen-Inaktivierung in Blutprodukten durch Bestrahlung, umfasst folgende Teile:
a) mindestens eine Strahlungsquelle, die UV-A Strahlung und/oder UV-B Strahlung und/oder UV-C Strahlung emittiert;
b) mindestens einen Strömungskanal, durch den das zu bestrahlende Blutprodukt strömt und mindestens eine Halterung, in die mindestens ein derartiger Strömungskanal reversibel oder irreversibel befestigt werden kann, wobei der mindestens eine Strömungskanal über mindestens eine Einlaßöffnung sowie mindestens eine Auslaßöffnung für das Blutprodukt verfügt, und der Durchmesser des mindestens einen Strömungskanals (**7**) in Richtung der Bestrahlung maximal der fünffachen, bevorzugt maximal der dreifachen und ganz besonders bevorzugt maximal der doppelten Eindringtiefe der verwendeten UV-Strahlung in das betreffende Blutprodukt entspricht;
c) ein Mittel zum Schutz des zu bestrahlenden Blutprodukts vor Überhitzung und
d) mindestens ein Fördermittel zur Förderung des zu bestrahlenden Blutprodukts.
Die mindestens eine Strahlungsquelle gemäß a) und der mindestens eine Strömungskanal und die mindestens eine Halterung gemäß b) sind so zueinander angeordnet, dass die verwendete UV-Strahlung mindestens 50%, bevorzugt mindestens 70%, besonders bevorzugt mindestens 90% des Innenvolumens des mindestens einen Strömungskanals, der entweder fest in der Vorrichtung montiert oder reversibel in der Halterung befestigt ist, erreicht, wenn dieser von der zu bestrahlenden Flüssigkeit durchströmt wird. Als dasjenige Innenvolumen eines Strömungskanals, das nach Möglichkeit wie vorstehend beschrieben bestrahlt werden soll, ist das Innere des Strömungskanals anzusehen, das sich innerhalb einer optionalen Einhäusung desjenigen räumlichen Bereichs der Vorrichtung befindet, der der UV-Strahlung ausgesetzt ist. Außerhalb dieses Bereiches befindliche Abschnitte des mindestens einen Strömungskanals unterliegen nicht der Bestrahlung. Die Querschnittsgeometrie des mindestens einen Strömungskanals kann sehr frei gewählt werden, ohne den Umfang der Erfindung oder ihrer Äquivalente zu verlassen. Hinsichtlich der Querschnittsgeometrie sind im wesentlichen quadratische, rechteckige, rundliche, ovale oder hohlzylinderartige Strömungskanal-Querschnitte bevorzugt. Wichtig ist, dass die darin strömende Flüssigkeit möglichst vollständig belichtet werden kann; dazu wird die Geometrie des mindestens einen Strömungskanals sowohl grundsätzlich als auch ggf. hinsichtlich Querschnittsgeometrie vorteilhafterweise an die Geometrie der mindestens einen Strahlungsquelle angepasst. Der Strömungskanal ist entweder flexibelwandig oder starrwandig aus einem Material ausgeführt, das für die verwendete UV-Strahlung durchlässig ist. Alternativ zu mindestens einem fest montierten Strömungskanal oder zusätzlich zu mindestens einem fest montierten Strömungskanal kann die erfindungsgemäße Vorrichtung auch mindestens eine Halterung aufweisen, in die mindestens ein Strömungskanal reversibel befestigt werden kann.

Dem Fachmann sind aus dem Stand der Technik zahlreiche Strahlungsquellen für UV-Strahlung bekannt, die einzeln oder in Kombination miteinander verwendet werden können, ohne den Umfang der Erfindung oder ihrer Äquivalente zu verlassen. Beispielsweise, jedoch nicht einschränkend, seien genannt: Quecksilber-Hochdrucklampen, Quecksilber-Niederdrucklampen, UV-LED's (also lichtemittierenden Dioden, die UV-Strahlung emittieren). Dem Fachmann ist bekannt, dass für den Betrieb dieser Strahlungsquellen diverse elektrische und/oder elektronische Vorrichtungen erforderlich sind, deren Einsatz mit der erfindungsgemäßen Verwendung mit involviert sind, aber keiner näheren Beschreibung im Rahmen der vorliegenden Offenbarung bedürfen, da dies dem Fachmann aus dem Stand der Technik bekannt ist. UV-LED's werden beispielsweise oftmals zu mehreren in einer entsprechenden Anordnung eingesetzt und gemeinsam elektronisch gesteuert.

Bei dem UV-durchlässigen Material für den mindestens einen Strömungskanal der Vorrichtung handelt es sich um ein Material, ausgewählt aus der Liste umfassend EVA, PMMA, PMP (Polymethylpenten), PVC, PE, Glas, Quarzglas.

Bei dem Fördermittel zur Förderung des zu bestrahlenden Blutproduktes handelt es sich beispielsweise um eine Schlauchpumpe oder eine Kolbenpumpe oder eine Kreiselpumpe. Bei medizinischen/pharmazeutischen Vorrichtungen werden oftmals vorteilhaft Schlauchpumpen eingesetzt, um steriles Einweg-/Einmalmaterial verwenden zu können. Dies ist auch bei der hier offenbarten Vorrichtung der Fall. Aber es ist natürlich auch erfindungsgemäß, Kolbenpumpen, etwa Spritzenpumpen, zu verwenden oder - bei Einbindung der Vorrichtung bzw. des Verfahrens in einen großtechnischen Produktionsprozeß - Kreiselpumpen. Die Vorrichtung und das Verfahren an sich sind nicht auf die Verwendung eines bestimmten Pumpentyps beschränkt. Lediglich die Natur der zu bestrahlenden Flüssigkeit, beispielsweise die Gegenwart mechanisch empfindlicher Zellen oder Zellbestandteile schränkt die Pumpenauswahl ggf. ein. Es ist vorteilhaft, das Fördermittel steuer- bzw. regelbar auszuführen, um die Strömungsgeschwindigkeit und damit die Belichtungsdauer gestuft oder stufenlos variieren zu können.

Es fällt ebenfalls in den Umfang der Vorrichtung sowie ihrer Äquivalente, wenn es sich bei dem Fördermittel zur Förderung des Blutproduktes um eine Anordnung handelt, bei der ein mit dem zu bestrahlenden Blutprodukt gefülltes erstes Vorratsgefäß über den mindestens einen Strömungskanal mit einem zweiten leeren Vorratsgefäß verbunden ist, wobei sich das erste Vorratsgefäß über dem zweiten Vorratsgefäß befindet, so dass die Förderung des zu bestrahlenden Blutproduktes durch die natürliche Schwerkraft als Antriebsmittel erfolgt. Hierbei handelt es sich um eine besonders bevorzugte Variante des Fördermittels, da sie äußerst kostengünstig und einfach ist. Es handelt sich um das im medizinischen Bereich universell einsetzbare Verfahren zum Entleeren gefüllter Infusionsbeutel jeglicher Art.

Bei dem Mittel zum Schutz des Blutproduktes vor Überhitzung handelt es sich um einen Lüfter, einen Ventilator oder ein Gebläse, mit dem kühlere Umgebungsluft oder ein anderes Fluid (z.B. Gas) durch die Vorrichtung geblasen wird, um den mindestens einen Strömungskanal mit dem darin strömenden Blutprodukt und/oder die Strahlungsquelle zu kühlen. Vom Umfang der Erfindung sowie ihrer Äquivalente umfasst ist beispielsweise auch die Verwendung von flüssigem Stickstoff oder Trockeneis, indem beispielsweise das jeweils abdampfende, bzw. absublimierende kühle Gas zur Kühlung verwendet wird.

Bei dem Mittel zum Schutz des Blutproduktes vor Überhitzung kann es sich vorteilhafterweise auch um ein Wasser-Kühlsystem, z.B. einen Umlaufthermostaten handeln, mit dem Kühlmedium durch die Vorrichtung geleitet wird, um den mindestens einen Strömungskanal mit dem darin strömenden Blutprodukt und/oder die Strahlungsquelle zu kühlen. Anstelle von Wasser können natürlich auch andere Wärmeträgermedien, etwa Öle oder Salzlösungen verwendet werden. Auch die Verwendung von Kältemaschinen unterschiedlichster Bauart (beispielsweise Kompressionskältemaschinen, Adsorptions-/Absorptions-kältemaschinen, Peltierelementen u. a. m) wird vom Umfang der Erfindung sowie ihrer Äquivalente umfasst.
Es fällt weiterhin in den Umfang der Erfindung, die mindestens eine Strahlungsquelle ausreichend weit von dem mindestens einen Strömungskanal entfernt zu positionieren und dadurch die Abwärme der mindestens einen Strahlungsquelle durch natürlicherweise im Betrieb einsetzende Konvektion abzuführen, sofern die mindestens eine Strahlungsquelle über eine ausreichend intensive Strahlkraft verfügt oder die emittierte Strahlung durch Reflektoranordnungen auf den mindestens einen Strömungskanal konzentriert werden kann, so dass trotz der größeren Entfernung zwischen Strahlungsquelle und Strömungskanal eine ausreichende Pathogen-Inaktivierung erreicht wird. Entsprechende bauliche Anordnungen bzw. Konstruktionen sind ebenfalls Mittel zum Schutz der zu bestrahlenden Flüssigkeit vor Überhitzung im Sinne dieser Offenbarung.

Natürlich fällt es in den Rahmen der Erfindung sowie Ihrer Äquivalente, das Mittel zum Schutz des zu bestrahlenden Blutproduktes mit einem Temperatursensor und/oder einer entsprechenden Recheneinheit (z. B. Mikroprozessor, Computer oder SPS-Modul (SPS = speicherprogrammierbare Steuerung)) zu koppeln, so dass die Temperatur des zu bestrahlenden Blutproduktes und/oder der Strahlungsquelle direkt oder indirekt gesteuert oder geregelt wird. Eine direkte Steuerung bzw. Regelung im Sinne dieser Offenbarung liegt dann vor, wenn der betreffende Temperatursensor die Temperatur der zu bestrahlenden Flüssigkeit selbst misst, eine indirekte Steuerung bzw. Regelung liegt vor, wenn die Temperaturmessung lediglich in der Nähe der Flüssigkeit erfolgt, und aus bekannten, evtl. experimentell bestimmten Zusammenhängen daraus ausreichend sicher auf die Temperatur der Flüssigkeit rückgeschlossen werden kann.

Die erfindungsgemäße Vorrichtung kann natürlich vorteilhafterweise zur Fokussierung der UV-Strahlung auf den mindestens einen Strömungskanal auch mindestens einen Reflektor für die verwendete UV-Strahlung aufweisen. Dies kann beispielsweise vorteilhaft sein, um Energie zum Betrieb der Vorrichtung einzusparen und/oder die Vorrichtung kompakter bauen zu können. Dem Fachmann sind aus dem Stand der Technik zahlreiche Reflektoren bzw. Reflektormaterialien bekannt, die verwendet werden können, ohne den Umfang der Vorrichtung sowie ihrer Äquivalente zu verlassen.

Vorteilhafterweise weist die erfindungsgemäße Vorrichtung auch eine Vorrichtung auf, um eine schwankende bzw. infolge Abnutzung nachlassende Strahlungsemission auszugleichen. Weiterhin kann es angebracht sein, eine Sicherheitsverriegelung vorzusehen, um ein unbeabsichtigtes Öffnen der Vorrichtung zu verhindern.

Ein wesentlicher Vorteil des Verfahrens besteht darin, dass zahlreiche (deutlich mehr als etwa 10) Dosiseinheiten (Spenderbeutel) gleichzeitig bearbeitet werden können, da aufgrund des geringen räumlichen Platzbedarfes eines Strömungskanales mehr separate Flüssigkeitsströme gleichzeitig bestrahlt werden können als fertig abgepackte Beutel. Fertig abgepackte Beutel sind sehr unhandlich, "sperrig", und es können daher mit den im Stand der Technik bekannten Belichtungsapparaturen nie mehr als etwa zehn Stück parallel bestrahlt werden.

Die Erfindung umfasst weiterhin die Verwendung des Verfahrens zur Inline-Pathogeninaktivierung im Zuge der Durchführung einer Apherese sowie eine entsprechende Vorrichtung. Das heißt, die Vorrichtung wird in Kombination mit einer Apheresemaschine oder als Bestandteil einer Apheresemaschine verwendet. Dies ist insbesondere dann sinnvoll, wenn die Erkrankung durch im Blutkreislauf zirkulierende Pathogene oder Pathogen-Vorstufen hervorgerufen bzw. begleitet wird, beispielsweise bei Malaria oder einer Blutvergiftung (Sepsis). Es ist naheliegend und fällt in den Umfang der Erfindung sowie Ihrer Äquivalente, dass die erfindungsgemäße Vorrichtung sowohl separat an die Apheresemaschine angeschlossen werden kann, als auch, dass sie baulich in die Apheresemaschine integriert ist. Weiterhin wird von der Erfindung und ihren Äquivalenten sowohl die Kombination der Patogen-Inaktivierung mit anderen Apherese-Maßnahmen umfasst als auch die Durchführung der Pathogen-Inaktivierung als einziger Maßnahme im Zuge der Apherese.

Weiterhin wird von der Erfindung natürlich auch ein Bausatz zum Bau einer Vorrichtung für die Durchführung des Verfahrens erfasst. Ein derartiger Bausatz umfasst mindestens eine Strahlungsquelle, mindestens einen Strömungskanal, mindestens eine Halterung für den mindestens einen Strömungskanal, ein Mittel zum Schutz des zu bestrahlenden Blutprodukes vor Überhitzung sowie optional mindestens ein Fördermittel zur Förderung des zu bestrahlenden Blutproduktes und/oder eine Bauanleitung zum Zusammenbau der Vorrichtung aus den Bestandteilen des Bausatzes, gegebenenfalls unter Verwendung von Werkzeugen sowie allgemein in Werkstatteinrichtungen üblicherweise vorhandenen Materialien und Methoden, die nicht in dem Bausatz enthalten sein müssen.

Ein weiterer Vorteil, den das erfindungsgemäße, sehr zeiteffiziente und mit wenig technischem Aufwand durchführbare Verfahren bietet, ist die mehrmals hintereinander erfolgende Behandlung von Proben (Blutprodukt-Einheiten), ähnlich einer sogenannten fraktionierten Sterilisierung. Dadurch ist es möglich, die Effizienz des Verfahrens noch weiter zu erhöhen. Es ist im Rahmen der Erfindung und ihrer Äquivalente auch möglich, die Pathogen-Inaktivierung nochmals als Sicherheit erst kurz vor der Anwendung des Blutproduktes oder während der Anwendung durchzuführen.

Die Vorrichtung sowie das Verfahren können auch vorteilhaft auch in der Pharmazeutischen Industrie als in den jeweiligen Prozeßstrom integriertes System zur Pathogeninaktivierung eingesetzt werden.

Nachfolgend wird nun die Erfindung unter Bezugnahme auf die beiliegenden Zeichnungen anhand einiger bevorzugter Ausführungsbeispiele näher beschrieben, ohne dass damit jedoch eine Einschränkung auf die gezeigten Beispiele verbunden ist. Insbesondere sind auch alle möglichen Merkmalskombinationen einzelner Merkmale der offenbarten Ausführungsbeispiele - unabhängig davon, ob sie explizit benannt werden oder nicht - vom Umfange der Erfindung sowie ihrer Äquivalente umfasst.

### Ausführungsbeispiele

In den begleitenden Zeichnungen bezeichnen gleiche Bezugszeichen jeweils gleiche/analoge technische Merkmale in den einzelnen Ausführungsbeispielen. Die Zeichnungen sind nicht maßstäblich ausgeführt, sondern skizzieren zur Verdeutlichung jeweils einzelne Merkmale der Vorrichtung. Aus dem Stand der Technik bekannte Teilvorrichtungen, etwa Kupplungssysteme zum Anschließen von Schläuchen und/oder Rohrleitungen, elektronische Bauteile, Steuerungssysteme, Medienversorgungsleitungen und dergleichen, die verwendet werden, ohne dadurch den Umfang der Erfindung oder ihrer Äquivalente zu verlassen, sind in den Zeichnungen der Übersichtlichkeit halber nicht dargestellt.

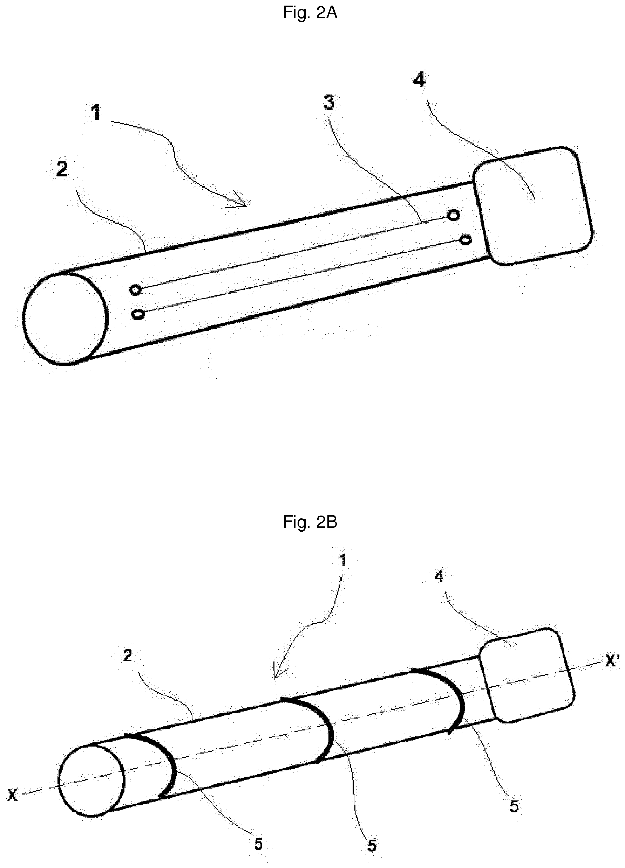

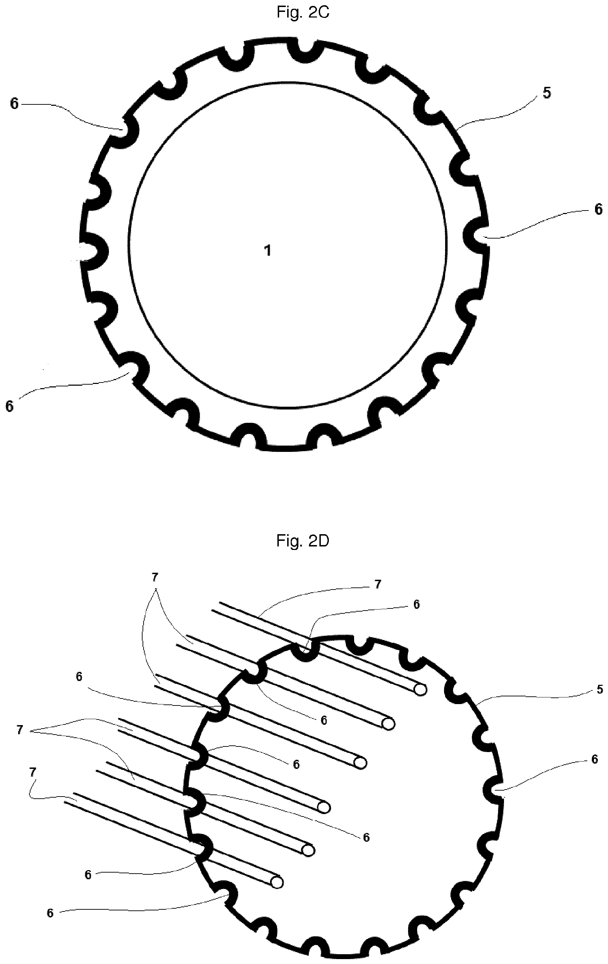

Die Zeichnungen Fig. 2A/B/C und D zeigen einzelne Bestandteile der Vorrichtung, schematisch dargestellt. Fig. 2A zeigt beispielhaft eine Strahlungsquelle für UV-C Strahlung **1**, umfassend eine Leuchtmittelabdeckung **2** (z. B. Quarzglasröhre), Leuchtmittel **3** sowie Elektrik **4** zum Betrieb der Strahlungsquelle. Bei dem Leuchtmittel **3** kann es sich beispielsweise um eine oder mehrere Quarzglasampullen mit eingeschmolzenen Elektroden handeln, in denen sich der bei Betrieb der Strahlungsquelle zur Lichtemission angeregte Quecksilberdampf befindet. In den folgenden Zeichnungen ist diese Strahlungsquelle **1** zum Zwecke der Übersichtlichkeit weiter vereinfachend ohne Leuchtmittel **3** dargestellt.

Fig. 2B zeigt in perspektivisch-schematischer Ansicht eine Strahlungsquelle **1** mit drei daran befindlichen Ringhalterungen **5**, die entfernbar oder nicht-entfernbar an der Strahlungsquelle befestigt sind, und an die ihrerseits einer oder mehrere Strömungskanäle entfernbar oder nicht-entfernbar befestigt werden können. Fig. 2C zeigt diese Anordnung schematisch skizziert im Querschnitt mit einer Schnittebene senkrecht zur Längsachse X-X' in Höhe einer Ringhalterung **5**. Es ist nicht zwingend erforderlich, genau drei Ringhalterungen **5** vorzusehen; es können auch weniger oder mehr sein, je nach gewünschter mechanischer Stabilität der Anordnung.

Man erkennt in Fig. 2C schematisch skizziert die Strahlungsquelle **1**, etwa eine UV-C Röhre, sowie eine Ringhalterung **5**, die die Strahlungsquelle **1** an der betreffenden Stelle teilweise oder vollständig (im konkreten Beispiel vollständig) umschliesst. Die Ringhalterung **5** kann form-, kraft- oder stoffschlüssig mit der Strahlungsquelle **1** verbunden sein. Es fällt darüber hinaus auch in den Umfang der Erfindung, wenn die Ringhalterung **5** keinen Kontakt mit der Strahlungsquelle **1** hat, sondern sie lediglich symmetrisch oder asymmetrisch umschliesst, etwa indem die Ringhalterung **5** (oder mehrere Ringhalterungen **5**) an der Innenseite einer oder mehreren Gehäusewänden (der Übersichtlichkeit halber nicht gezeigt) befestigt ist. In diesem Falle ist es vorteilhaft, wenn die Befestigungsvorrichtungen **6** an Ringhalterung **5** (vgl. folgenden Absatz) nicht nach radial nach außen weisen, wie in Fig. 2C gezeigt, sondern radial nach innen.

Die mindestens eine Ringhalterung **5** weist mindestens eine Befestigungsvorrichtung **6** auf zur Befestigung mindestens eines Strömungskanals **7**, beispielsweise in Form eines Schlauches. Dem Fachmann sind zahlreiche Befestigungsvorrichtungen **6** bekannt, mit Hilfe derer runde oder eckige schlauch- oder rohrförmige Strukturen, z. B. Kuststoffschläuche, Installationsrohre, Stromleitungen Besenstiele, Stangen allgemein u. a. m. reversibel oder nicht-reversibel an Flächen wie z. B. Wänden befestigt werden können. Alle derartigen Strukturen, geeignet an der Ringhalterung **5** befestigt oder darin einkonstruiert, sind erfindungsgemäß verwendbar. Als darin einkonstruiert sind zum Beispiel Befestigungsstrukturen **6** anzusehen, wenn sie zum Beispiel als flexible Einkerbungen mit oder ohne Hinterschneidungen in einem Stück gemeinsam mit der Ringhalterung **5** zum Beispiel aus einem Polymerwerkstoff gefertigt sind. Geeignete Arten der Befestigung der Befestigungsvorrichtungen **6** an der Ringhalterung **5** umfassen beispielsweise Kleben, mechanisch Verklemmen, Nageln, Nieten, Schrauben u.a. Die jeweilige genaue Konstruktion und Wirkweise der Befestigungsvorrichtungen **6** ist für die Funktionalität der erfindungsgemäßen Vorrichtung nicht relevant. Daher und der Vielfalt der erfindungsgemäß möglichen Arten von Befestigungsvorrichtungen **6** geschuldet, sind sie in den Abbildungen lediglich schematisch, beispielsweise als rundliche Einkerbungen, bzw. Einbuchtungen, oder auch anders dargestellt.

In Fig. 2D ist schematisch skizziert dargestellt, wie mehrere Strömungskanäle **7** in Befestigungsvorrichtungen **6** einer Ringhalterung **5** befestigt ("eingeklinkt") sind. Der Vollständigkeit halber sei an dieser Stelle darauf hingewiesen, dass in den Zeichnungen der Übersichtlichkeit halber mehrfach vorkommende Merkmale nicht immer mit dem zugehörigen Bezugszeichen versehen sind, sondern nur teilweise. Aus den sehr schematisch gehaltenen Zeichnungen geht für den Fachmann anhand der beispielhaft angegebenen Bezugszeichen eindeutig hervor, welche der weiteren gezeigten, jedoch nicht mit Bezugszeichen versehenen Merkmale zu welchem Bezugszeichen gehören.

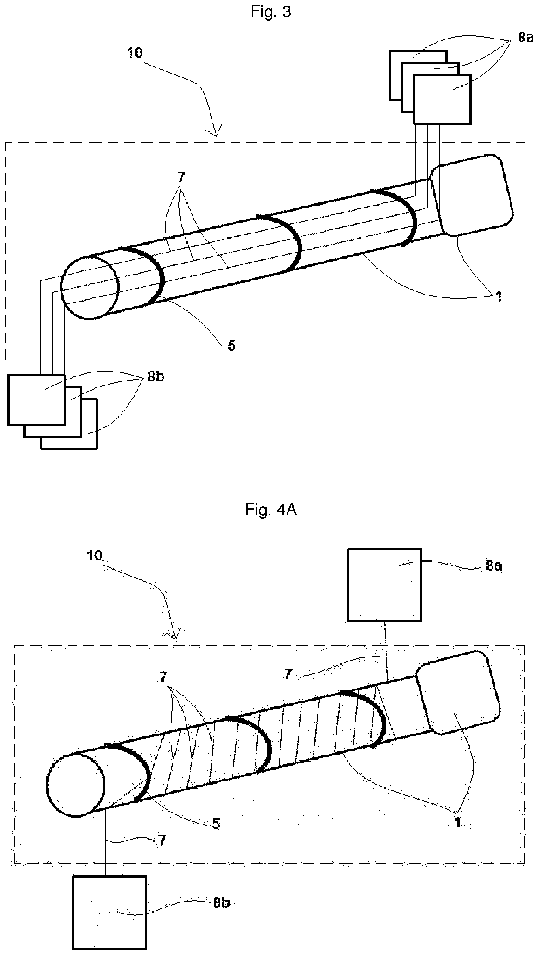

Fig. 3 zeigt schematisch beispielhaft eine erste Ausführungsform der erfindungsgemäßen Vorrichtung **10**, umfassend eine Strahlungsquelle **1** mit drei Ringhalterungen **5** sowie mehrere Strömungskanäle **7**, angedeutet durch die strichlierte Umrandung, wobei jeder der Strömungskanäle **7** an den Enden mit jeweils einem Vorratsbeutel **8a**, **8b** für Blutprodukte verbunden ist. Der Übersichtlichkeit halber sind die Anschlüsse der Strömungskanäle **7** an die Vorratsbeutel **8a**, **8b**, d.h. die mindestens eine Einlaßöffnung sowie die mindestens eine Auslaßöffnung, nicht dargestellt; beliebige aus dem Stand der Technik bekannte Anschlußarten können verwendet werden, ohne den Umfang der Erfindung sowie ihrer Äquivalente zu verlassen. Sowohl in diesem als auch in den weiteren Ausführungsbeispielen sind Fördervorrichtungen für die zu bestrahlende Flüssigkeit (die ebenfalls in den Zeichnungen der Übersichtlichkeit halber nicht dargestellt ist) nicht dargestellt, um damit anzudeuten, dass es auch erfindungsgemäß ist, eine Höhendifferenz in einem Schwerkraftfeld zwischen dem an dem einen Ende eines Strömungskanals 7 angeschlossenen Vorratsbeutel **8a** und dem am anderen Ende des Strömungskanals angeschlossenen Vorratsbeutel **8b** als Fördermittel für die zu bestrahlende Flüssigkeit zu verwenden, sofern sich der die unbehandelte Flüssigkeit enthaltende zunächst gefüllte Vorratsbeutel **8a** oberhalb des zunächst leeren, die behandelte Flüssigkeit aufnehmende Vorratsbeutel **8b** befindet. In derartigen Fällen kann die Strömungsgeschwindigkeit vorteilhafterweise mittels Quetschhahn oder dergleichen am - in diesem Falle flexibelwandigen - Strömungskanal **7** reguliert werden. Auch die Wahl der Höhendifferenz zwischen **8a** und **8b** kann zur Einstellung der Strömungsgeschwindigkeit herangezogen werden.

Fig. 3 zeigt somit eine Ausführungsform der erfindungsgemäßen Vorrichtung, mit der es möglich ist, zahlreiche Flüssigkeits-Einzelportionen gleichzeitig entlang der Strahlungsquelle **1** zu führen und somit zu bestrahlen.

Fig. 4A zeigt schematisch beispielhaft dargestellt eine zweite Ausführungsform der erfindungsgemäßen Vorrichtung **10**, bei der ein Strömungskanal **7** um eine röhrenförmige Strahlungsquelle **1** herum gewickelt ist, um die strömende Flüssigkeit bei gleicher Strömungsgeschwindigkeit intensiver bestrahlen zu können, indem sie für einen längeren Zeitraum der UV-Strahlung ausgesetzt wird. Diese Ausführungsvariante kann vorteilhafterweise auch als inline-System in einer pharmazeutischen Produktionslinie eingesetzt werden. Auch hier enthält der Vorratsbeutel **8a** zunächst unbehandeltes Produkt (unbehandelte Flüssigkeit), und der Vorratsbeutel **8b** dient dazu, das bestrahlte Produkt (die bestrahlte Flüssigkeit) aufzunehmen. Es ist natürlich auch möglich, mehrere Strömungskanäle **7** gleichzeitig (z.B. nebeneinander) um eine Strahlungsquelle **1** herumzuwickeln und somit mehrere voneinander unabhängige Produktströme gleichzeitig zu belichten.

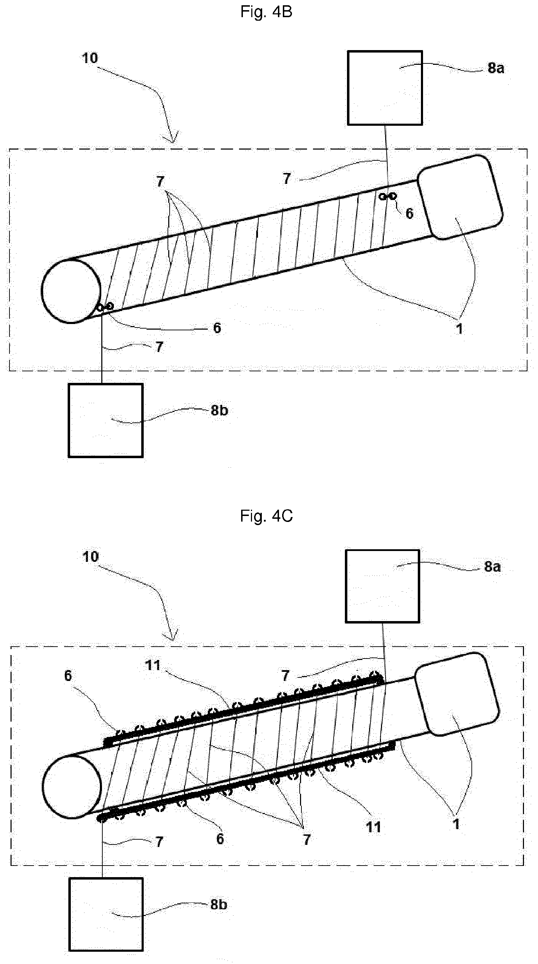

Fig. 4B zeigt schematisch beispielhaft dargestellt eine dritte Ausführungsform der erfindungsgemäßen Vorrichtung **10**, die eine Variante der in Fig. 4A gezeigten Ausführungsform ist. Hierbei werden keine Ringhalterungen **5** verwendet, die ergänzend zur bisher beschriebenen Verwendung auch dafür verwendet werden können, den Strömungskanal **7 -** vergleichbar der Verwendung von Gummiringen - festzuklemmen, sondern der Strömungskanal **7** wird mit Hilfe von zwei oder mehreren Befestigungsvorrichtungen **6** so direkt an der Strahlungsquelle **1** fixiert, dass die sich dazwischen befindlichen Windungen des Strömungskanals **7** ausreichend sicher fixiert sind. Aus dem Stand der Technik sind zahlreiche dazu geeignete Befestigungsvorrichtungen **6**, z. B. Schrauben, Haken, Ösen, Klemmen, Klebepunkte usw. bekannt, die erfindungsgemäß verwendet werden können. Darüber hinaus gilt für die in Fig. 4B gezeigten Befestigungsvorrichtungen **6** auch das bereits erläuternd zu Fig. 2C hinsichtlich Befestigungsvorrichtungen **6** Gesagte analog. Die Befestigungsvorrichtungen **6** können in der Ausführungsform gemäß Fig. 4B natürlich - analog zur Ausführungsform gemäß Fig. 2C (dort indirekt über die Ringhalterung **5**) - auch an einer oder mehreren Gehäusewänden (nicht gezeigt) befestigt sein. In Verbindung mit einem Strömungskanal **7** mit ausreichender Stabilität erhält man so einen schraubenfederartig angeordneten Strömungskanal **7**, bei dem die Strahlungsquelle **1** in den Hohlraum des schraubenfederartig angeordneten Strömungskanals **7** eingeschoben werden kann. Es ist für den Fachmann ohne weiteres einsichtig, dass in dieser Ausführungsform die Befestigungsvorrichtungen **6** gleichzeitig die Aufgabe der Ringhalterungen **5** sowie der Klemmleisten **11** in den anderen Ausführungsbeispielen übernimmt (die Positionierung/Befestigung), so daß Ausführungsformen wie in Fig. 4B keine zusätzlichen Ringhalterungen **5** oder Klemmleisten **11** benötigen. Der Begriff "Befestigungsvorrichtung" **6** ist daher in den entsprechenden Fällen zusätzlich funktional auch als "reduzierte" Ringhalterung **5** bzw. "reduzierte" Klemmleiste **11** zu verstehen. Um diesen Sachverhalt und auch weitere Möglichkeiten der Fixierung von Strömungskanälen **7** (s.u.) übergeordnet zu erfassen, wird der allgemeine Begriff "Halterung" verwendet, der die kennzeichnenden Merkmale **5**, **6**, **11**, **12a**, **12b** umfasst.

Fig. 4C zeigt schematisch beispielhaft dargestellt eine vierte Ausführungsform der erfindungsgemäßen Vorrichtung **10,** die ebenfalls eine Variante der in Fig. 4A gezeigten Ausführungsform ist. Hierbei werden anstelle von Ringhalterungen **5** Klemmleisten **11** verwendet, um den Strömungskanal **7** zu fixieren. Die Klemmleisten **11** weisen ebenfalls jeweils eine oder mehrere Befestigungsvorrichtungen **6** auf, funktionieren in der gleichen Weise wie die Ringhalterungen **5** und können auch aus dem gleichen Material gefertigt sein, sind jedoch nicht ringförmig ausgebildet, sondern stabförmig. Sie umfassen die Strahlungsquelle somit nicht radial senkrecht zur Achse X-X', sondern sind an oder neben der Strahlungsquelle 1 parallel zu dieser, also in Richtung der Achse X-X' befestigt und erlauben es in besonders vorteilhafter Weise, schraubenfederartig gewickelte Strömungskanäle **7** zu fixieren. Die Befestigung der Klemmleisten **11** kann auf jeder der bekannten Weisen erfolgen, wie sie auch für die Ringhalterungen **5** und die Befestigungsvorrichtungen **6** erfindungsgemäß sind. Es ist erfindungsgemäß, eine oder mehrere derartige Klemmleisten **11** zu verwenden, und die Klemmleisten **11** können ebenfalls in analoger Weise entweder an der Strahlungsquelle **1** oder an einer oder mehreren Wänden (der Übersichtlichkeit halber nicht gezeigt) befestigt werden.

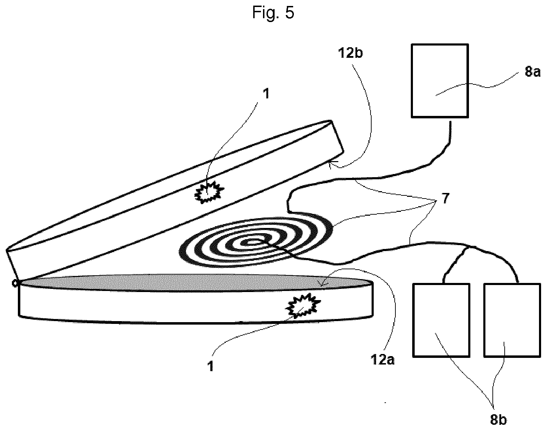

Fig. 5 zeigt schematisch beispielhaft dargestellt eine fünfte Ausführungsform der erfindungsgemäßen Vorrichtung **10**, bei der der Strömungskanal **7** spiralförmig zusammengerollt zwischen zwei im wesentlichen flächig ausgeführten Strahlungsquellen **1** angeordnet ist, so dass der Kanalquerschnitt größer gewählt werden kann, da die Bestrahlung von zwei Seiten erfolgt. Neben einer spiralförmigen Anordnung sind natürlich noch weitere Anordnungen des mindestens einen Strömungskanals **7** möglich, etwa mäanderförmig oder kammartig auffächernd, d.h. aus einem Eingangsbereich des Strömungskanals zweigen mehrere Seitenkanäle ab und führen in separate Auffangbeutel (entspr. Vorratsbeutel **8b**). Die Aufspaltung eines ursprünglichen Gesamt-Flüssigkeitsstromes in mehrere Teilströme kann natürlich auch nach erfolgter Bestrahlung erfolgen, wie in Fig. 5 angedeutet ist. Der Vorteil der Ausführungsform gemäß Fig. 5 ist, dass die Aufgabe der Ringhalterung **5**, bzw. der Klemmleisten **11** und der Befestigungsvorrichtungen **6** durch die planen Auflage- bzw. Andruckflächen **12a/12b** der Strahlungsquellen **1** wahrgenommen wird, so dass der sprialig oder mäanderförmig angeordnete Strömungskanal **7** im zusammengeklappten Zustand der Vorrichtung automatisch optimal zu der mindestens einen Strahlungsquelle **1** ausgerichtet und fixiert ist. Vorteilhafterweise ist der mäanderartig oder spiralig gewundene Strömungskanal **7** bereits von sich aus in dieser Form fixiert, so dass nur die Enden flexibel positioniert und an die entsprechenden Vorratsbeutel **8a**/**8b** angeschlossen werden müssen. Die Auflagefläche **12a** sowie die Andruckfläche **12b** können auch senkrecht oder in einem beliebigen anderen Winkel zur Horizontalen angeordnet sein, wobei dann ggf. beide als Andruckflächen fungieren. **12a** und **12b** sind beispielsweise aus UV-durchlässigem Quarzglas gefertigt. Auflage- und Andruckfläche **12a**/**12b** müssen nicht zwingend durch ein Scharnier miteinander verbunden sein, wie es in Fig. 5 schematisch angedeutet ist.

Wie bereits vorstehend an zahlreichen Ausführungsvarianten erläutert, kann die Befestigung - oder allgemeiner: Lagerung - des mindestens einen Strömungskanals **7** sehr variabel ausgestaltet werden, ohne den Umfang der Erfindung oder ihrer Äquivalente zu verlassen, beispielsweise auch, indem der mindestens eine Strömungskanal **7** einfach nur in beliebiger Anordnung, jedoch ausreichender Länge auf einer beliebigen mehr oder weniger horizontalen Oberfläche ausgelegt und mit UV-Strahlung bestrahlt wird, während die zu bestrahlende Flüssigkeit (Blutprodukt) hindurchströmt. Alternativ kann er beispielsweise auch auf einem geeigneten Drahtgestell ausgelegt und von oben oder von unten, oder von oben und unten (sowie ggf. auch von der Seite) bestrahlt werden.

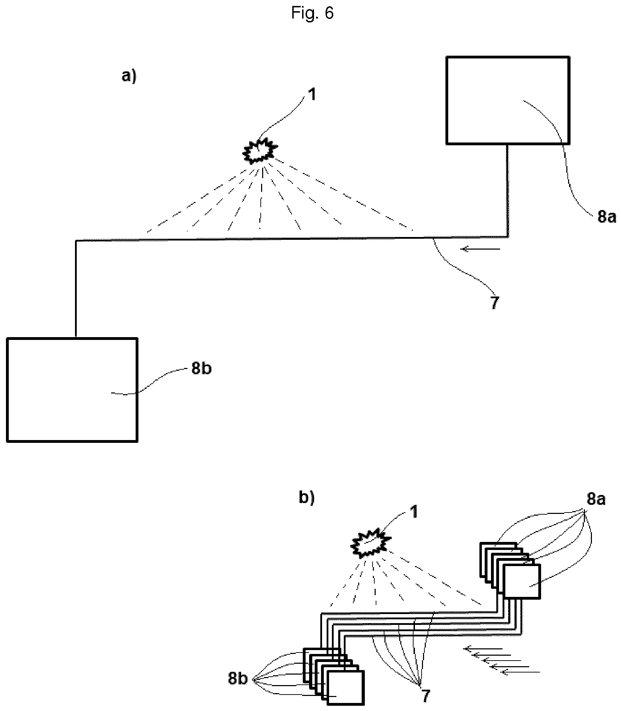

In Fig. 6 sind nochmals die Verfahrensvarianten der Einzelchargen-Bestrahlung (Bestrahlung eines einzelnen Produktes, einer einzelnen Spendereinheit (a)) und der parallelen Bestrahlung zahlreicher Einzelchargen (b) sehr stark schematisiert einander gegenübergestellt. (Die Strömung der Flüssigkeit wird durch Pfeile symbolisiert, die Bestrahlung mit UV-Strahlung durch unterbrochene Linien, ausgehend von der Strahlungsquelle **1** in Richtung Strömungskanal/Strömungskanäle **7**). Das Verfahren ist sehr flexibel einsetzbar und in einem weiten Bereich skalierbar. Beide Varianten können sowohl in großtechnische Fertigungsprozesse integriert, als auch individuell in kleinem Maßstab betrieben werden.

Alle vorstehend beispielhaft genannten Ausführungsformen der erfindungsgemäßen Vorrichtung haben den weiteren Vorteil, keinen Größenbeschränkungen zu unterliegen. Es ist jedoch von besonderem Vorteil, wenn die Vorrichtung so klein und kompakt ausgeführt ist, dass sie von einer Person transportiert und auf einem Tisch abgestellt werden kann. Die Vorrichtung gemäß Fig. 5 weist z. B. vorteilhafterweise eine Grundflächen von maximal etwa 20 cm x 30 cm auf.

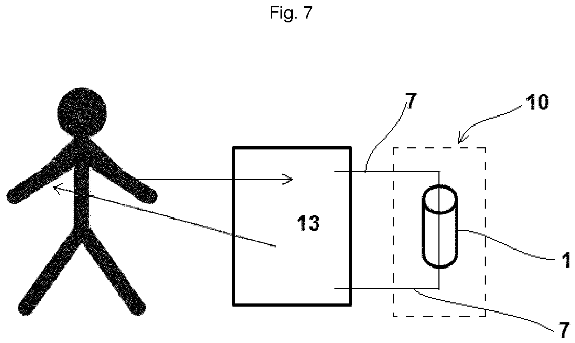

In Fig. 7 ist schematisch skizziert die Verwendung des Verfahrens zur Inline-Pathogeninaktivierung im Zuge der Durchführung einer Apherese dargestellt. Anstelle von Spenderblut bzw. Spender-Blutprodukten wird das Eigenblut des Patienten extrakorporal bestrahlt, um darin enthaltene Pathogene zu inaktivieren, und ihm anschließend zurück infundiert (skizziert durch den zu dem Patienten hin sowie den von ihm weg gerichteten Pfeil). Beispielhaft dargestellt ist in Fig. 7 die Ausführungsvariante, dass eine erfindungsgemäße Vorrichtung **1** über den Strömungskanal 7 separat an eine bestehende Apheresemaschine **13** angeschlossen ist, so dass die erfindungsgemäße Pathogen-Inaktivierung ergänzend zu weiteren bekannten Apherese-Maßnahmen vorgenommen wird. An dieser Stelle sei ergänzend angemerkt, dass in Fig. 7 schematisch skizziert eine Ausführungsform der Vorrichtung **10** dargestellt ist, bei der die Strahlungsquelle **1** hohlzylinderförmig ausgebildet ist, und der Strömungskanal **7** durch diese hohlzylinderartige Strahlungsquelle hindurchgeführt wird, so dass der Strömungskanal **7** von allen Seiten bestrahlt wird.

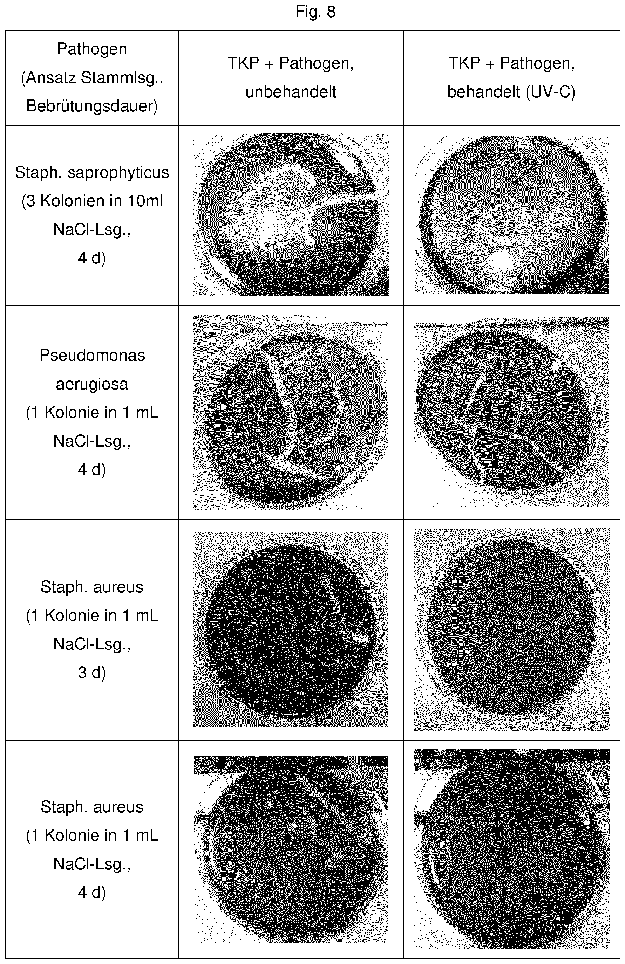

In Fig. 8 sind beispielhaft einige Versuchsergebnisse zur Wirksamkeit des erfindungsgemäßen Verfahrens gegenüber ausgewählten Pathogenen unter Verwendung von gepooltem Thrombozytenkonzentrat (TKP) dargestellt. Dazu wurde jeweils ein in bekannter Weise hergestelltes TKP aus je vier Spendereinheiten mit einem Pathogen angeimpft, anschließend in zwei Teilproben aufgeteilt und die eine Teilprobe dem erfindungsgemäßen Verfahren unterzogen. Danach wurde in jeder der beiden Teilproben der Pathogen-Titer durch Ausstreichen der TKP-Teilproben auf geeigneten Nährböden und Bebrüten bestimmt. Dem Fachmann sind die dazu erforderlichen Schritte sowie die zu verwendenden Nährböden und Bebrütungsparameter bekannt. Die für die Beimpfung verwendete Pathogen-Stammlösung wurde gewonnen, indem jeweils eine oder mehrere Pathogen-Kulturen aus entsprechenden Anzuchtplatten entnommen und in isotonischer Kochsalzlösung suspendiert wurden. Von der so erhaltenen Pathogen-Stammlösung wurde dem TKP zur Beimpfung jeweils 1 mL zugesetzt. Weitere Versuchsdetails können Tab. 2 (Fig. 8) entnommen werden. Aus den in Fig. 8 dargestellten Ergebnissen ist die Effektivität des erfindungsgemäßen Verfahrens klar ersichtlich: Man erkennt deutlich, daß pathogen-infizierte, unbestrahlte, TKP-Proben bei Inkubation auf Nährböden sehr starkes Pathogenwachstum zeigen, infizierte und anschließend bestrahlte TKP-Proben, jedoch sehr viel weniger.

### Abbildungslegenden

- Fig. 1:: Tabelle 1 - Beispielhaft zusammengestellte Inaktivierungszeiten für Mikroorganismen in wässriger Phase aus dem Stand der Technik, die erfindungsgemäß für die Durchführung des Verfahrens ausgewählt werden können.
- Fig. 2A:: Strahlungsquelle **1** für UV-C Strahlung (schematisch)
- Fig. 2B:: Perspektivisch-schematische Teilansicht einer Strahlungsquelle **1** mit drei daran befindlichen Ringhalterungen **5**.
- Fig. 2C:: Querschnitt durch eine Strahlungsquelle 1 mit Ringhalterung **5** in einer Schnittebene senkrecht zur Längsachse X-X' (Fig. 2B) in Höhe einer Ringhalterung **5**.
- Fig. 2D:: Schematisch skizzierte Darstellung einer teilweise mit Strömungskanä-len 7 bestückten Ringhalterung **5**.
- Fig. 3:: Schematisch beispielhaft dargestellte erste Ausführungsform der erfin-dungsgemäßen Vorrichtung **10**, die die gleichzeitige Bestrahlung meh-rerer Flüssigkeitschargen (Applikationsportionen) ermöglicht.
- Fig. 4A:: Schematisch beispielhaft dargestellte zweite Ausführungsform der er-findungsgemäßen Vorrichtung **10**, bei der beispielhaft ein Strömungs-kanal **7** um eine röhrenförmige Strahlungsquelle **1** herum gewickelt ist.
- Fig. 4B:: Schematisch beispielhaft dargestellte dritte Ausführungsform der erfin-dungsgemäßen Vorrichtung **10**, bei der beispielhaft ein Strömungskanal **7** um eine röhrenförmige Strahlungsquelle **1** herum gewickelt und lediglich an den Enden befestigt ist.
- Fig. 4C:: Schematisch beispielhaft dargestellte vierte Ausführungsform der erfindungsgemäßen Vorrichtung **10**, bei der beispielhaft ein Strömungskanal **7** um eine röhrenförmige Strahlungsquelle **1** herum gewickelt und mittels geeigneter Klemmleisten **11** befestigt ist.
- Fig. 5:: Schematisch beispielhaft dargestellte fünfte Ausführungsform der erfindungsgemäßen Vorrichtung **10**, bei der der Strömungskanal **7** spiralförmig zusammengerollt zwischen zwei im wesentlichen flächig ausge-führten Strahlungsquellen **1** angeordnet ist.
- Fig. 6:: Schematische Übersicht der beiden grundsätzlichen Verfahrensvarian-ten.
- Fig. 7:: Schematisch skizzierte Darstellung der Verwendung des Verfahrens zur Inline-Pathogeninaktivierung im Zuge der Durchführung einer Apherese.
- Fig. 8:: Tabelle 2 - Versuchsergebnisse einiger beispielhaft durchgeführten Versuche zur Wirksamkeit des erfindungsgemäßen Verfahrens.

### Bezugszeichenliste

- **1**: Strahlungsquelle
- **2**: Leuchtmittelabdeckung
- **3**: Leuchtmittel
- **4**: Elektrik
- **5**: Ringhalterung
- **6**: Befestigungsvorrichtung für einen Strömungskanal 7
- **7**: Strömungskanal
- **8a**: Vorratsbeutel, anfänglich enthaltend die zu bestrahlende Flüssigkeit
- **8b**: Vorratsbeutel zur Aufnahme der bestrahlten Flüssigkeit
- **10**: Erfindungsgemäße Vorrichtung
- **11**: Klemmleiste
- **12a**: Auflage, bzw. Andruckfläche
- **12b**: Andruck, bzw. Auflagefläche

## Patentansprüche

1. Vorrichtung (**10**) zur Inaktivierung von Pathogenen in Blutprodukten durch Bestrahlung, **dadurch gekennzeichnet, dass** die Vorrichtung (**10**) folgende Teile umfasst:
a) mindestens eine Strahlungsquelle (**1**), die UV-A Strahlung und/oder UV-B Strahlung und/oder UV-C Strahlung emittiert;
b) mindestens einen im wesentlichen quadratischen, rechteckigen, rundlichen, ovalen oder hohlzylinderartigen Strömungskanal (**7**), der entweder flexibelwandig oder starrwandig aus einem Material ausgeführt ist, das für die verwendete UV-Strahlung durchlässig ist, und mindestens eine Halterung (**5**, **6**, **11**, **12a**, **12b**), in die mindestens ein derartiger Strömungskanal (7) reversibel oder irreversibel befestigt werden kann, wobei der mindestens eine Strömungskanal (7) über mindestens eine Einlaßöffnung sowie mindestens eine Auslaßöffnung für das Blutprodukt verfügt, und der Durchmesser des mindestens einen Strömungskanals (**7**) in Richtung der Bestrahlung maximal der fünffachen, bevorzugt maximal der dreifachen und ganz besonders bevorzugt maximal der doppelten Eindringtiefe der verwendeten UV-Strahlung in das betreffende Blutprodukt entspricht;
c) ein Mittel zum Schutz des zu bestrahlenden Blutprodukts vor Überhitzung und
d) mindestens ein Fördermittel zur Förderung des zu bestrahlenden Blutprodukts,
wobei die mindestens eine Strahlungsquelle (**1**) gemäß a) und der mindestens eine Strömungskanal (**7**) und die mindestens eine Halterung (**5**, **6**, **11**, **12a**, **12b**) gemäß b) so zueinander angeordnet sind, dass die verwendete UV-Strahlung mindestens 50%, bevorzugt mindestens 70%, besonders bevorzugt mindestens 90% des Innenvolumens des mindestens einen Strömungskanals (**7**) erreicht, wenn dieser von dem Blutprodukt durchströmt wird.

2. Vorrichtung (**10**) gemäß Anspruch 1, **dadurch gekennzeichnet, dass** es sich bei der Strahlungsquelle (**1**) um eine Quecksilber-Hochdrucklampe und/oder eine Quecksilber-Niederdrucklampe und/oder um eine betriebsbereite Anordnung von einer oder mehreren lichtemittierenden Dioden handelt, die UV-Strahlung emittieren.

3. Vorrichtung (**10**) gemäß mindestens einem der vorstehenden Ansprüche, **dadurch gekennzeichnet, dass** es sich bei dem UV-durchlässigen Material um ein Material handelt, ausgewählt aus der Liste umfassend EVA, PMMA, PMP, PVC, PE, Glas, Quarzglas.

4. Vorrichtung (**10**) gemäß mindestens einem der vorstehenden Ansprüche, **dadurch gekennzeichnet, dass** es sich bei dem Fördermittel zur Förderung des Blutproduktes um eine Schlauchpumpe oder eine Kolbenpumpe oder eine Kreiselpumpe handelt.

5. Vorrichtung (**10**) gemäß mindestens einem der vorstehenden Ansprüche 1 bis 3, **dadurch gekennzeichnet, dass** sie zusätzlich mindestens ein erstes Vorratsgefäß (**8a**) und mindestens ein weiteres zweites Vorratsgefäß (**8b**) umfasst, wobei es sich bei dem Fördermittel zur Förderung des zu bestrahlenden mindestens einen Blutproduktes um eine Anordnung handelt, bei der das mit dem zu bestrahlenden Blutprodukt gefüllte erste Vorratsgefäß (**8a**) über den Strömungskanal (**7**) mit dem zweiten leeren Vorratsgefäß (**8b**) verbunden ist, wobei sich das erste Vorratsgefäß (**8a**) über dem zweiten Vorratsgefäß (**8b**) befindet, so dass die Förderung des zu bestrahlenden Blutproduktes durch die natürliche Schwerkraft als Antriebsmittel erfolgt.

6. Vorrichtung (**10**) gemäß mindestens einem der vorstehenden Ansprüche, **dadurch gekennzeichnet, dass** es sich bei dem Mittel zum Schutz des zu bestrahlenden Blutproduktes vor Überhitzung um einen Lüfter, einen Ventilator oder ein Gebläse handelt, mit dem kühlere Umgebungsluft oder ein anderes Fluid durch die Vorrichtung geblasen wird, um den mindestens einen Strömungskanal (**7**) mit der darin strömenden Flüssigkeit und/oder die Strahlungsquelle (**1**) zu kühlen.

7. Vorrichtung (**10**) gemäß mindestens einem der vorstehenden Ansprüche 1 bis 5, **dadurch gekennzeichnet, dass** es sich bei dem Mittel zum Schutz des zu bestrahlenden Blutproduktes vor Überhitzung um ein Wasser-Kühlsystem, z.B. einen Umlaufthermostaten handelt, mit dem Kühlmedium durch die Vorrichtung geleitet wird, um den mindestens einen Strömungskanal (**7**) mit der darin strömenden Flüssigkeit und/oder die Strahlungsquelle (**1**) zu kühlen.

8. Vorrichtung (**10**) nach einem der Ansprüche 6 oder 7, **dadurch gekennzeichnet, dass** das Mittel zum Schutz des zu bestrahlenden Blutproduktes vor Überhitzung mit einem Temperatursensor und/oder einer entsprechenden Recheneinheit gekoppelt ist, so dass die Temperatur der zu bestrahlenden Flüssigkeit und/oder der Strahlungsquelle (**1**) direkt oder indirekt gesteuert oder geregelt wird.

9. Vorrichtung (**10**) gemäß mindestens einem der vorstehenden Ansprüche, **dadurch gekennzeichnet, dass** die Vorrichtung (**10**) zur Fokussierung der UV-Strahlung auf den mindestens einen Strömungskanal (**7**) mindestens einen Reflektor für die verwendete UV-Strahlung aufweist.

10. Verwendung einer Vorrichtung (**10**) gemäß mindestens einem der vorstehenden Ansprüche in Kombination mit einer Apheresemaschine oder als Bestandteil einer Apheresemaschine.

11. Bausatz zum Bau einer Vorrichtung (**10**) gemäß mindestens einem der vorstehenden Ansprüche 1 bis 9, umfassend
- mindestens eine Strahlungsquelle (**1**),
- mindestens einen Strömungskanal (**7**), und mindestens eine Halterung (**5**, **6, 11, 12a, 12b**) für mindestens einen Strömungskanal (**7**),
- ein Mittel zum Schutz des zu bestrahlenden Blutproduktes vor Überhitzung
- sowie optional
o mindestens ein Fördermittel zur Förderung des zu bestrahlenden mindestens einen Blutproduktes und/oder
o eine Bauanleitung zum Zusammenbau der Vorrichtung (**10**) aus den Bestandteilen des Bausatzes.

12. Verfahren zur Inaktivierung von Pathogenen in Blutprodukten, **dadurch gekennzeichnet, dass** das zu bestrahlende Blutprodukt an mindestens einer UV-Strahlungsquelle in einer variablen oder konstanten Schichtdicke entlanggeführt und bestrahlt wird, die maximal der fünffachen, bevorzugt maximal der dreifachen und ganz besonders bevorzugt maximal der doppelten Eindringtiefe der verwendeten UV-Strahlung in das betreffende Blutprodukt entspricht, und die Fließgeschwindigkeit des Blutproduktes so eingestellt, gesteuert oder geregelt wird, dass eine Bestrahlungszeit von 0,01 s bis 1000 s, bevorzugt von 0,1 s bis 100 s und besonders bevorzugt von 1 s bis 100 s erreicht wird.

13. Verfahren gemäß Anspruch 12, **dadurch gekennzeichnet, dass** es sich bei der verwendeten UV-Strahlung um UV-A Strahlung und/oder UV-B Strahlung und/oder UV-C Strahlung handelt.

14. Verfahren gemäß einem der vorangehenden Ansprüche 12 bis 13, **dadurch gekennzeichnet, dass** es sich bei dem Blutprodukt um ein Blutprodukt handelt, ausgewählt aus der Liste umfassend Vollblut, Thrombozytenkonzentrat, Blutplasma, Erythrozytenkonzentrat, Granulozytenkonzentrat, autologe Serum-Augentropfen, allogene Serum-Augentropfen oder eine andere aus Vollblut gewonnene Flüssigkeit.
